# EUROPEAN PATENT APPLICATION

(11) **EP 4 361 253 A1**
(43) Date of publication of application: **01.05.2024**
(21) Application number: 22828485.7
(22) Date of filing: 23.06.2022
(51) Int. Cl.: C12N 5/07, C12N 1/00, C12N 1/08, C12P 1/00

(54) **DECELLULARIZED CELL STRUCTURE**

(30) Priority: 23.06.2021 JP 2021103763
(71) Applicant: ADEKA CORPORATION, Arakawa-ku Tokyo 116-8554 (JP)
(72) Inventor: MUTO, Mio, Tokyo 116-8554 (JP); HIWATARI, Kenichiro, Tokyo 116-8554 (JP); KUMAGAI, Hironori, Tokyo 116-8554 (JP)
(74) Representative: Gille Hrabal Partnerschaftsgesellschaft mbB Patentanwälte
(86) International application number: PCT/JP2022/025023
(87) International publication number: WO 2022/270569

(57) **Abstract**

The object of the present invention is to provide a decellularized graft which has any thickness and specific shape.

The problem can be solved by a decellularized cellular structure of the present invention, which is a decellularized cultured cellular structure, characterized by having porosity ratio of 30% to 80%.

## Description

### TECHNICAL FIELD

The present invention relates to a decellularized cellular structure.

### BACKGROUND ART

When transplanting a graft derived from a living tissue of another person or another species of animal, graft rejection by the recipient tissue is problematic. In order to improve the compatibility of grafts with biological tissues, techniques have been developed to use decellularized tissues consisting of the remaining supportive tissues (extracellular matrix, ECM) after removal of cells from biological tissues as grafts. Decellularization means the removal of cellular components such as nucleic acids that are antigenic to the recipient, thereby avoiding immune rejection (Patent literatures 1 and 2).

### CITATION LIST

### PATENT LITERATURE

[Patent literature 1] JP 2005-514971 A
[Patent literature 2] WO 2016/136633
[Patent literature 3] JP 2012-139541 A
[Patent literature 4] JP 2014-148321 A
[Patent literature 5] JP 2018-051415 A
[Patent literature 6] JP 2004-357694 A
[Patent literature 7] JP 2020-198794 A
[Patent literature 8] JP 4517125 B

### SUMMARY OF INVENTION

### TECHNICAL PROBLEM

The decellularized tissues are useful in that decellularization of living tissues yields grafts that are free from rejection. However, in some cases, a stacking process is required to obtain a certain thickness thereof. In addition, it was not easy to fabricate a specific shape. Further, it was not easy to maintain the strength of the graft.

Therefore, the object of the present invention is to provide a decellularized graft which has any thickness and maintain hardness.

### SOLUTION TO PROBLEM

The present inventors have conducted intensive studies into a decellularized graft having any thickness and specific shape, as a result, surprisingly found that the decellularized graft having any thickness and maintaining hardness can be obtained by decellularizing cultured cellular structures (Patent literatures 3 to 7) obtained by culture.

The present invention is based on the above findings.

Therefore, the present invention relates to:
[1] a decellularized cellular structure, which is a decellularized cultured cellular structure, characterized by having porosity ratio of 30% to 80%,
[2] the decellularized cellular structure of the item [1], wherein a hardness of the decellularized cellular structure is 0.20 to 5.00 (N),
[3] the decellularized cellular structure of the item [1] or [2], wherein a DNA content in dry mass is 0.020 % by weight or less with respect to the decellularized cellular structure,
[4] the decellularized cellular structure of the item [1] or [2], comprising a protein with (a) molecular weight of 45,000 to 53,000 and (b) isoelectric point of pI 4.00 to 4.90,
[5] the decellularized cellular structure of the item [4], wherein the protein is tubulin,
[6] a method for preparing a decellularized cellular structure, comprising: a formation step of cultured cellular structure wherein the cultured cellular structure is formed by culturing cells, and a decellularization step wherein the cultured cellular structure is decellularized to adjust porosity ratio of 30% to 80%,
[7] the method for preparing a decellularized cellular structure of the item [6], wherein the cells are cultured in an any shape before or simultaneously with the formation of the cultured cellular structure in the formation step of cultured cellular structure,
[8] the method for preparing a decellularized cellular structure of the item [7], wherein the culture of cells in the any shape before the formation of the cultured cellular structure in the formation step of cultured cellular structure, is sheet-cell culture or cellular spheroid culture,
[9] the method for preparing a decellularized cellular structure of the item [6], wherein after the formation step of cultured cellular structure, the cells are cultured in an any shape,
[10] the method for preparing a decellularized cellular structure of any one of items [6] to [9], wherein the decellularization is performed by high hydrostatic pressure treatment, and
[11] The method for preparing a decellularized cellular structure of any one of items [6] to [9], wherein a thickness retention ratio before and after decellularization in the decellularization step is 90% or more.

### ADVANTAGEOUS EFFECTS OF INVENTION

According to the decellularized cellular structure of the present invention, the decellularized graft having any thickness and maintaining hardness can be provided. In the preparing method of the present invention, a thickness after decellularization with respect to the thickness before decellularization can be maintained, and a shape before and after decellularization can be maintained. That is to say, a decellularized material having any thickness can be provided, and whereby a decellularized material having specific shape can be provided, and the decellularized materials can be made according to the intended use, such as grafts or cell therapy. Further, decellularized materials with strength and without rejection can be produced.

### BRIEF DESCRIPTION OF THE DRAWINGS

[Fig. 1] Fig. 1 is a graph showing porosity ratios of the decellularized cellular structures of Examples 1 to 4 and Comparative Example 1.
[Fig. 2] Fig. 2 is a graph showing thickness retention ratios of the decellularized cellular structures of Examples 1 to 4 and Comparative Example 1.
[Fig. 3] Fig. 3 is a micrograph of the decellularized cell structure which is Sirius red-stained.
[Fig. 4] Fig. 4 is a graph showing results of hardness tests of the decellularized cellular structures of Examples 1 to 4 and Comparative Example 1.
[Fig. 5] Fig. 5 is micrographs showing protein spots of Example 1 (A) and Comparative Example 1 by two-dimensional electrophoresis.

### DESCRIPTION OF EMBODIMENTS

### [1] Decellularized cellular structure

The decellularized cellular structure of the present invention is a decellularized cultured cellular structure, and porosity ratio thereof is 30% to 80%.

### «Cultured cellular structure»

The cultured cellular structures in the present invention are cellular structures obtained by in vitro culture. The cultured cellular structure is not particularly limited, as long as the cells produce extracellular matrix (ECM) extracellularly, and whereby the cells adhere to each other and the three-dimensional shape is maintained. The smallest distance (thickness) in three dimensions is preferably 50 µm or more, and more preferably 100 µm or more. The size in the other two dimensions is also not particularly limited, but is, for example, 1 mm or more, and preferably 6 mm or more, and whereby the decellularized cellular structures can be efficiently prepared. This enables efficient preparation of decellularized cellular structures.

The strength of the cultured cell structure is not particularly limited as long as it maintain a certain three-dimensional shape, but preferably it has the strength enough to be handled with tweezers or sutured in wet conditions.
The shape of the cultured cell structure is not particularly limited, but for example, there may be mentioned a sheet type, a tube type, or a block type. And whereby, it is easy to create decellularized cellular structures according to the intended use, such as grafts or cell therapy.

The decellularized cellular structures can be provided in a wet or dry state. The decellularized cellular structures provided in the wet state can be used immediately for transplantation. On the other hand, the decellularized cellular structures provided in the dry state can be stored for a long period of time, and can be easily transported. Then, it can be restored and used at the time of use.

Cells are roughly classified into floating cells and adherent cells. Cells for cultured cell structures are not limited, but preferably are adherent cells from the viewpoint of ease of preparation of cultured cell structures.

The adherent cells are not particularly limited, but any type of cells can be selected according to the purpose of use of the cultured structure. For example, differentiated adherent cells include hepatocytes, astrocytes, Kupffer cells, vascular endothelial cells, sinusoidal endothelial cell, endothelial cells, fibroblasts, osteoblasts, osteoclasts, periodontal ligament derived cells, epidermal cells, tracheal epithelial cells, gastrointestinal epithelial cells, cervical epithelial cells, epithelial cells, mammary gland cells, pericytes, smooth muscle cells, cardiac muscle cells, myocytes, renal cells, pancreatic islet cells of Langerhans, peripheral nerve cells, neurons, chondrocytes, or osteocytes. For example, undifferentiated adherent cells include embryonic stem cells (ES cells), embryonic germ cells (EG cells), germline stem cells (GS cells), induced pluripotent stem cells (iPS cells), mesenchymal stem cells, hematopoietic stem cells, neural stem cells, cardiac progenitor cells, vascular endothelial progenitor cells, neural progenitor cells, adipose progenitor cells, dermal fibroblasts, skeletal muscle myoblasts, osteoblasts, or odontoblasts. The dermal fibroblasts, mesenchymal stem cells, and cardiac progenitor cells are preferrable from the viewpoint of ease of preparation of cultured cell structures.

An origin of cell is not particularly limited in origin, and the cells include eukaryotic cells, prokaryotic cells, multicellular organism cells, or unicellular organism cells. Eukaryotic cells include animal cells, insect cells, plant cells, fungi, algae, or protozoa. The animal cell is not limited, but includes cells derived from human, monkey, dog, cat, rabbit, ferret, sheep, goat, cow, pig, horse, camel, mouse, rat, hamster, guinea pig, or gerbil.

The cultured cellular structure preferably is attached by the extracellular matrix, although not limited thereto. Adhesion by the extracellular matrix provides a certain strength to the cultured cell structure. As extracellular matrices, there may be mentioned, for example, collagen, laminin, fibronectin, chondroitin sulfate, heparin sulfate, keratan sulfate, hyaluronic acid, or the like. However, they vary depending on the cell types, and thus they are not particularly limited thereto.

### <<DNA content>>

The DNA content per dry mass of the decellularized cellular structure is 0.020% by mass or less, preferably 0.015% by mass or less, more preferably 0.010% by mass or less, and even more preferably 0.008% by mass or less, even more preferably 0.006% by mass or less, even more preferably 0.004% by mass or less, even more preferably 0.002% by mass or less, even more preferably 0.0018% by mass or less, even more preferably 0.0015% by mass or less, and whereby it is possible to obtain a decellularized cellular structure which is properly decellularized and has less rejection when transplanted.

The DNA content can be measured by the PicoGreen method. A dried specimen of the decellularized cellular structure (hereinafter referred to as "sample") is immersed in proteolytic enzyme solution to dissolve the same. Then, it is treated with phenol/chloroform to remove proteins, and DNA is recovered by ethanol precipitation. The recovered DNA is fluorescently stained with PicoGreen (Life Technologies) and the DNA is quantified by measuring the fluorescence intensity, and the DNA content (mass) of the sample is calculated. For quantification, a calibration curve prepared using the standard DNA provided with Picogreen is used. From the dry mass and DNA content of the sample, the DNA ratio is calculated according to the following formula. (DNA content per dry mass (hereinafter sometimes referred to as decellularized DNA ratio)) = (DNA content of dried specimen of decellularized cellular structure)/(mass of dried specimen of decellularized cellular structure)

### <<Porosity ratio>>

The porosity ratio of the decellularized cellular structure of the present invention is 30% to 80%. The lower limit of porosity ratio is preferably 33%, more preferably 37%, further preferably 40%, further more preferably 42%, even more preferably 44%, further even more preferably 47%, and most preferably 50%. The upper limit of porosity ratio is preferably 77%, more preferably 73%, further preferably 70%, further more preferably 68%, even more preferably 66%, further even more preferably 64%, and most preferably 55%. The grafts with sufficient strength and without rejection can be obtained by the above ranges. In addition, it is also possible to provide the decellularized graft having any thickness or specific shape.

In the present specification, the porosity ratio is calculated as follows. A section of decellularized cellular structure is stained with Sirius red, which stains extracellular matrix. The microscopic tissue image is analyzed by image processing software, and the areas not stained by Sirius red are considered as pores, and the porosity ratio is calculated.

Sirius red staining was performed as follows. The section sample of the decellularized cellular structure is deparaffinized. Next, the section sample is treated with iron hematoxylin. Then, the section sample is treated with a hydrochloric acid/ethanol solution, and is treated with phosphomolybdic acid aqueous solution. Next, it is treated with Sirius Red (Direct Red 80), dehydrated, and sealed.

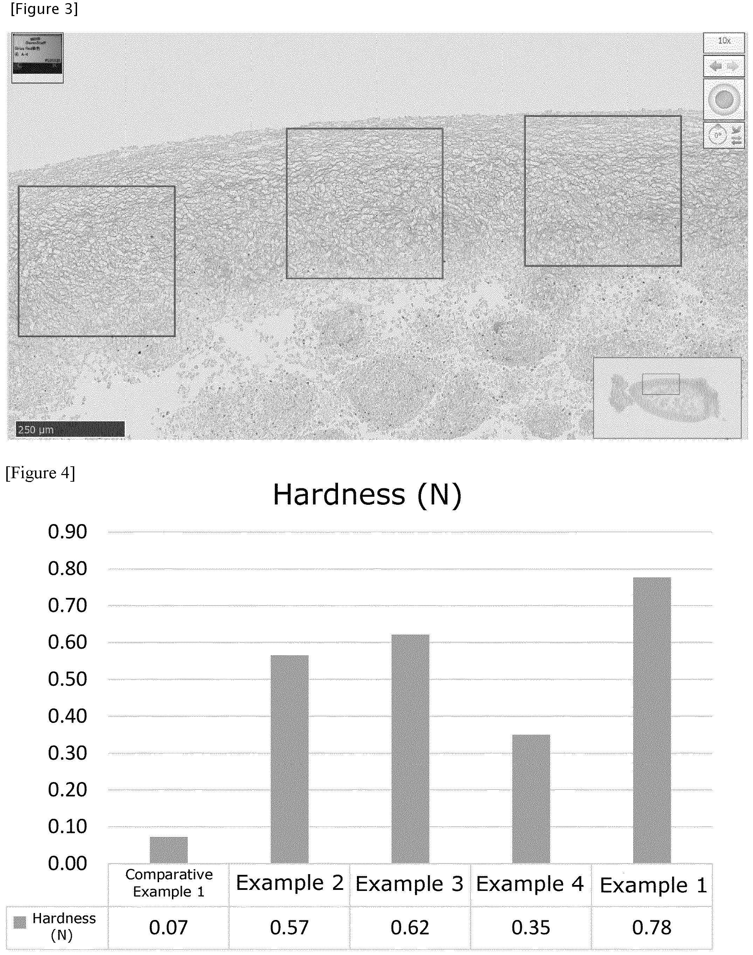

The obtained Sirius red stained sections are observed with a microscope and the porosity ratio is calculated using image processing software (ImageJ). The sections are photographed at 10x magnification and the area of the entire decellularized cellular structure and the area stained by Sirius red are measured respectively (Figure 3). the porosity ratio is calculated by the following formula. Porosity ratio (%)=(Area of entire decellularized cellular structure - Area stained by Sirius red) / Area of entire decellularized cellular structure × 100

### [2] Method for preparing decellularized cellular structure

The method for decellularized cellular structure of the present invention comprises a formation step of cultured cellular structure wherein the cultured cellular structure is formed by culturing cells, and a decellularization step wherein the cultured cellular structure is decellularized to adjust porosity ratio of 30% to 80%.

### <<Formation step of cultured cellular structure (1)>>

In the formation step of cultured cellular structure (1), the cultured cellular structure is formed by culturing cells. The cells used are not particularly limited, as long as the cells can be cultured in vitro. For example, there may be mentioned primary cells directly collected from tissues or organs, or passage cells in which the primary cells directly collected from tissues or organs are passaged in several generations. In addition, passage cells that have been passaged for a long period of time may be used. Specifically, the cells described in the above "[1] Decellularized cellular structure " can be used without limitation.

The cultured cellular structures can be formed as follows. The cells produce extracellular matrix (ECM) outside the cells, and three-dimensional shape is maintained by adhesion and bonding between cells. The method for forming cultured cellular structure is not particularly limited, as long as the adhesion by extracellular matrix is used, and for example, the methods described in Patent literatures 3 to 8 can be used. Specifically, there may be mentioned the method for producing a sheet-like 3D structure without using a scaffold (Patent literature 3), the method for stacking multiple cell sheets (Patent literature 4), the method for producing a structure by supplying cellular spheroids to a support in which a mesh-like space is formed by multiple threads or needle-like members (Patent literature 5), the method for culturing cellular spheroids in a chamber (Patent literature 6), the method for producing a 3D cell structure by culturing cellular spheroids in a culture mold (Patent literature 7), or the method for producing a 3D cell structure by culturing cellular spheroids with a support comprising a thread-like body or needle-like body through which the cellular spheroids can be penetrated (Patent literature 8). In all of these methods, the extracellular matrix secreted from cells is used to form cellular structures by cell-cell adhesion. The extracellular matrix includes, but is not limited to, those described in the above"[1] Decellularized cellular structure."

For example, cellular spheroids (spheroids) can be cultured as follows to form cultured cellular structures. The appropriate culture medium can be selected according to the cells to be cultured. The cellular spheroid is placed in a cavity surrounded by reticular or comb-like supports with gaps smaller than the size of the cellular spheroid. The cavity is formed into a desired shape by the reticular or comb-like supports and plates, and the supports have many openings. The medium can be brought into contact with the cellular spheroids through these openings. While maintaining the cellular spheroids in the cavity, a container for shaking culture is shaken, and whereby the medium is effectively brought in to contact with the cell spheroids and the cellular spheroids are cultured well. The cellular spheroids produce the extracellular matrix, and the cellular spheroids begin to adhere to each other, and the cultured cellular structure with a desired three-dimensional shape can be produced.

In the formation step of cultured cellular structure (1), for example, the cells may be cultured into an any shape before or simultaneously with the formation of the cultured cellular structure. Culturing to an any shape means culturing to an any thickness or planar view shape. For example, the cells may be cultured to any planar view shape by adjusting the area. The cells of any planar view shape may be stacked, and cultured by adjusting any thickness and shape. The cultured cells may be cut into desired shapes. In the formation step of cultured cellular structure (1), the cells may be cultured to form any shape at the same time as the cultured cellular structure is formed. For example, the cells may be cultured to form an any shape at the same time as the method for forming the cultured cellular structure described above is performed. For example, cultured cellular structure can be formed by culturing cells in a gel containing extracellular matrix in three dimensions (3D).

The cells may be cultured in any shape before forming the cultured cellular structure.
In the formation step of cultured cellular structure, culturing cells in any shape before forming cultured cellular structures is preferably, for example, culturing cells in sheet form or culturing cellular spheroids. As the culturing cells before forming cultured cellular structure, for example, there may be mentioned culturing cells in an any planar view shape, or stacking a plurality of cell sheets (sheet-like cells) as described in Patent literature 4, and culturing these stacked cell sheets before forming cultured cellular structure. The cell sheet culture can be performed according to the method of cell sheet preparation using a culture plate or the like, which is usually practiced in this field. By stacking and culturing the obtained cell sheets, cells can be cultured to any thickness and shape. Then, the cells secrete extracellular matrix, and the stacked cell sheets adhere to each other to obtain the cultured cellular structure that maintains a three-dimensional shape.

Furthermore, as the culture before forming the cultured cellular structure, there may be mentioned, for example, that cell spheroids is produced by culture before cell adhesion, as described in Patent literature 5 or 7. Adherent cells cannot survive for a long period of time when they are floating in solution. When the adherent cells are placed in a non-adherent environment, they adhere to each other in search of scaffolds and form cell spheroids.

Specifically, the cellular spheroid can be prepared by aggregating multiple cells. After cells are cultured in a monolayer, they are transferred to water-repellent or non-adherent round-bottomed multi-wells or U-shaped plate (dimple plate) and incubated, and whereby cells are aggregated to form cellular spheroids. From the viewpoint of efficiency, an incubation time to form cell spheroids is preferably 6 to 48 hours, more preferably 6 to 24 hours. However, the method for preparing cellular spheroids is not limited to the above methods, and the gyratory culture method in which cell suspensions are placed in a gyrating solution, the method in which cell suspensions are placed in test tubes and precipitated by a centrifuge, the arginate bead method, or the preparation methods described in Patent literature 5, 6 or 8, can be used. From the viewpoint that a large amount of uniform cellular spheroids can be processed, the method of placing cell suspensions in water-repellent or cell-nonadherent multi-wells is efficient and preferrable.

By adjusting the incubation time of cellular spheroids, the cellular spheroid can be cultured to any desired thickness.
In addition, by adhering cellular spheroids together, the cellular spheroids can be cultured to any thickness and shape. By further culturing the obtained cellular spheroids, the cells secrete extracellular matrix, the cells adhere to each other, and cultured cellular structures maintaining a three-dimensional shape can be obtained. In addition, by culturing cellular spheroids in a cavity as described above, cultured cell structures with a desired three-dimensional shape can be precisely prepared.

The amount of extracellular matrix (ECM) when the cells are cultured into any shapes before forming cultured cellular structure, is preferably less than the amount of ECM when cultured cellular structures are formed.

After the formation step of cultured cellular structure, the cells may be cultured into an any shape. For example, after the above method for forming cultured cellular structures is performed, the cells may be further cultured to an any shape, or the cells may be coated with an extracellular matrix and then stacked and cultured to an any shape.

The amount of extracellular matrix (Extracellular matrix, ECM) when the cells are cultured into any shape after the formation step of cultured cellular structures is preferably the same or more than the amount of ECM when cultured cellular structures are formed.

### «Decellularization step (2)»

In the decellularization step (2), the cellular structure is decellularized to adjust porosity ratio of 30% to 80%. That is, the porosity ratio of the cellular structure after decellularization is 30% to 80%. The lower limit of porosity ratio is preferably 33%, more preferably 37%, further preferably 40%, further more preferably 42%, even more preferably 44%, further even more preferably 47%, and most preferably 50%. The upper limit of porosity ratio is preferably 77%, more preferably 73%, further preferably 70%, further more preferably 68%, even more preferably 66%, further even more preferably 64%, and most preferably 55%. The grafts with sufficient strength and without rejection can be obtained by the above ranges. In addition, it is also possible to provide the decellularized graft having any thickness or specific shape.

In the decellularization step, conventional methods can be used. The method of decellularization is not particularly limited as long as the effect of the present invention is obtained. However, there may be mentioned, for example, a hydrostatic pressure treatment, freezing and thawing method, ultrasonic treatment, enzyme treatment, treatment with hypertonic electrolyte solution, physical agitation, hypertonic solution/hypotonic solution method, enzyme treatment with proteolytic enzymes, nucleolytic enzyme, etc., treatment with alcohol solvents, or the like, and a combination of two or more of these methods may be used. In order to efficiently obtain the decellularized cellular structure and to achieve the effect of the present invention, the method by pressure processing is preferable.

When the decellularized structure is obtained by the high hydrostatic pressure treatment, hydrostatic pressure of 50 to 1500 MPa is applied to the obtained cellular structure in the medium. From the viewpoint of sufficient decellularization, the applied hydrostatic pressure is preferably 50 MPa or more. From the viewpoint of not requiring a pressure vessel that can withstand the pressurization, not requiring much energy, and preventing the cellular structure from being damaged by ice formation when the medium used for the application is a water-based medium, the applied hydrostatic pressure is preferably 1500 MPa or less. The applied hydrostatic pressure of 80 to 1300MPa is more preferable, 90 to 1200MPa is even more preferable, 95 to 1100MPa is even more preferable, 95 to 700MPa is even more preferable, and 400 to 700MPa is most preferable, from the viewpoint of the decellularization effect, bacteriostatic effect and virus inactivation effect, and ease of pressurization.

The media used for hydrostatic pressure application include water, saline solution, injection water, propylene glycol or its aqueous solution, glycerin or its aqueous solution, sugar aqueous solution, or the like. Buffer solutions include acetate buffer, phosphate buffer, citrate buffer, borate buffer, tartrate buffer, Tris buffer, HEPES buffer, or the like. These media may contain surfactants.

The temperature of the high hydrostatic pressure treatment is not particularly limited as long as ice does not form and heat does not damage the cellular structure. The temperature is preferably 0 to 45°C, more preferably 4 to 37°C, and most preferably 5 to 35°C, because the decellularization process is smoothly performed and the effect on the cellular structure is minimal. If the duration of the high hydrostatic pressure treatment is too short, the cells are not sufficiently destroyed, and if the duration of the high hydrostatic pressure treatment is too long, energy is wasted. Therefore, the duration to maintain the desired applied pressure in the high hydrostatic pressure treatment is preferably 1 to 120 minutes, more preferably 5 to 60, and even more preferably 7 to 30.

The cellular structure treated with high hydrostatic pressure is preferably treated with nucleolytic enzymes. The nucleolytic enzyme removes nucleic acid components from the hydrostatically treated cellular structure, and is not particularly limited to, for example, DNase (such as DNase I, DNase II) derived from pancreas, spleen, or E. coli.

The nucleolytic enzyme can be added to the medium (such as water, saline solution, injection solution, or buffer solution) used for the above high hydrostatic pressure treatment, to make the enzyme work. The amount of enzyme to be added depends on the type of enzyme and the definition of the number of units (U), but can be set appropriately by a person skilled in the art. In the case of DNase I, for example, 50~200 U/mL may be used. The treatment temperature also depends on the nucleolytic enzyme to be used, but for example, may be set at 1°C to 40°C. The treatment time is not particularly limited, but for example, may be set to 1 to 120 hours (preferably 1 to 96 hours, more preferably 1 to 48 hours). In case of low temperatures, the treatment may be long, and in case of high temperatures, the treatment time may be short.

The cellular structure treated with high hydrostatic pressure is washed with a washing liquid. The washing liquid may be the same as or different from the medium used for the high hydrostatic pressure treatment. The washing liquids preferably contain organic solvents or chelating agents. Organic solvents can improve the removal efficiency of lipids, and chelating agents can inactivate calcium and magnesium ions in the decellularized cellular structure, thereby preventing calcification when the particulate decellularized cellular structure of the present invention is applied to diseased areas. As organic solvents, water-soluble organic solvents are preferable because they are effective in removing lipids, and ethanol, isopropanol, acetone, and dimethyl sulfoxide are preferable. As chelating agents, there may be mentioned iminocarboxylic acid chelating agents, such as ethylenediaminetetraacetic acid (EDTA), nitrilotriacetic acid (NTA), diethylenetriamine pentaacetic acid (DTPA), hydroxyethylenediaminetriacetic acid (HEDTA), triethylenetetramine hexaacetic acid (TTHA), 1,3-propanediaminetetraacetic acid (PDTA), and 1,3-diamino-2-hydroxypropane tetraacetic acid (DPTA-OH), hydroxyethyl iminodiacetic acid (HIDA), dihydroxyethylglycine (DHEG), glycol ether diamine tetraacetic acid (GEDTA), dicarboxymethylglutamic acid (CMGA), 3-hydroxy-2, 2'-iminodisuccinic acid (HIDA), dicarboxymethyl aspartate (ASDA) or their salts; hydroxycarboxylic chelating agents, such as citric acid, tartaric acid, malic acid and lactic acid, or their salts. The salts of these chelating agents include sodium or potassium salts.

The washing temperature is not particularly limited as long as there is no heat damage to the cellular structure. The washing temperature is preferably 0 to 45°C, more preferably 1 to 40°C, and most preferably 2 to 35°C, since the washing property is good and there is little effect on the cellular structure. When washing, the washing liquid may be shaken or stirred as necessary.

When the decellularized cellular structure is obtained by the freezing and thawing method, the cellular structure is frozen at a temperature of -80 to -20°C (preferably -80 to -40°C) for 1 to 48 hours (preferably 10 to 30 hours), and then thawed at a temperature of 20 to 37°C. The above process is repeated once or more than twice (preferably 2 to 5 times). Then, the cellular structure is preferably treated with nucleolytic enzymes in the same way as the high hydrostatic pressure treatment. The cells in the frozen and thawed cellular structures are destroyed, and these cells are removed by the washing liquid. The washing method is the same as that in the high hydrostatic pressure treatment.

When the decellularized cellular structure is obtained by the ultrasonic treatment, the cellular structure is treated with ultrasonic waves (for example, intensity: 10W/cm², frequency: 10 kHz, duration: 2minutes), for example, in a saline solution. Then, the cellular structures are preferably shaken with a surfactant solution (such as 1 mass% Triton X (polyoxyethylene octylphenyl ether) solution) at 2 to 10°C (preferably 4°C) for 1 to 120 hours (preferably 12 to 120 hours). Then, the cellular structure is preferably treated with nucleolytic enzymes in the same way as the high hydrostatic pressure treatment. Then, it is preferably washed in the same way as the high hydrostatic pressure treatment.

The obtained decellularized cellular structure is preferably freeze-dried, although this is not a limitation. Freeze-drying can be omitted depending on the site of the cellular structure. In addition, the decellularized cellular structures may be sterilized by gamma irradiation, UV irradiation, or the like, and the sterilization by gamma irradiation is preferable.

### <<Thickness retention ratio>>

In the method for preparing decellularized cellular structure, the thickness can be maintained before and after decellularization in the decellularization step (2).
The thickness retention ratio before and after decellularization is not limited, but is, for example, 90% or more more preferably 92% or more and even more preferably 94% or more.
The thickness retention ratio can be calculated as follows. Thickness retention ratio(%)=(thickness of decellularized cellular structure/thickness of cultured cellular structure before decellularization)×100

### <<Hardness test>>

The hardness of the decellularized cellular structure of the present invention is not particularly limited as long as the effect of the present invention is obtained, but is, for example, 0.20 to 5.00(N). The lower limit of hardness is preferably 0.30(N), more preferably 0.40(N), further preferably 0.50(N), further more preferably 0.60(N), even more preferably 0.65(N), further even more preferably 0.70(N), and most preferably 0.73(N ), and most preferably 0.73(N). The upper limit of hardness is preferably 3.00(N), more preferably 1.00(N), further preferably 0.95(N), further more preferably 0.90(N), even more preferably 0.87(N), further even more preferably 0.83(N), and most preferably 0.80(N). The grafts with more sufficient strength and without rejection can be obtained by the above ranges. It is easy to handle when the decellularized cellular structure of the present invention is implanted as a graft, such as by picking it up with tweezers or piercing it with a surgical needle for suture. In addition, decellularized grafts with any thickness or specific shape can be provided.

In the present invention, the hardness is measured by Texture Profile Analysis (TPA) as follows. It is measured at room temperature by a texture analyzer TA.XT plus (EKO INSTRUMENT CO., LTD) equipped with a P/4 cylindrical probe (4 mm in diameter). The point at which a load of 0.01(N) is applied to the probe is set as the zero point, and the probe is compressed twice on a distance of 0.5mm at a rate of 0.02mm/sec. The maximum force (N) at the first compression cycle is determined as the hardness.

### <<Protein>>

The decellularized cellular structure of the present invention is not particularly limited as long as the effect of the present invention is obtained. However, for example, it comprises a protein with (a) molecular weight of 45,000 to 53,000 and (b) isoelectric point of pI 4.00 to 4.90. Whereby the effect of the present invention is further exhibited.

The molecular weight and isoelectric point (pI) of the above proteins can be determined, for example, by two-dimensional electrophoresis of the proteins and the above proteins can be identified by mass spectrometry.

In two-dimensional electrophoresis, either isoelectric point separation or molecular weight separation can be performed first in the first dimension. However, from the viewpoint of increasing the accuracy of measurement, it is preferable to perform isoelectric point separation in the first dimension and molecular weight separation in the second dimension. Two-dimensional electrophoresis can be performed according to the conventional method, and commercially available kits and apparatuses can be used. For example, isoelectric electrophoresis is performed using capillary gels or strip gels as separation media, and after the gel swimming is completed, molecular weight separation can be performed by electrophoresis in the direction perpendicular to the direction of expansion of isoelectric electrophoresis using a planar gel (such as SDS-polyacrylamide gel). The presence or absence of protein, molecular weight, and isoelectric point can be confirmed by staining the gel after two-dimensional electrophoresis according to the conventional method.

The molecular weight of the protein is preferably 45,000 to 53,000, more preferably 47,000 to 51,000, and even more preferably 50,000 to 50,500, from the viewpoint of exhibiting the effect of the present invention. The isoelectric point (pI) of protein A is preferably 4.00 to 4.90, more preferably 4.40 to 4.85, and even more preferably 4.60 to 4.80.

The protein is preferably a tubulin, more preferably alpha-tubulin or beta-tubulin, further preferably beta-tubulin. In humans, there are five types of tubulin: α (alpha), β (beta), γ (gamma), δ (delta), and ε (epsilon). Tubulin is highly conserved among species, forming heterodimers and further multimerizing to form microfilaments. A heterodimers of α-tubulin and β-tubulin is the basic structural units of microtubules involved in cell division. α-tubulin and β-tubulin has a molecular weight of about 55,000 and are similar to each other.

At least seven β-tubulin isotypes have been identified in humans. These isotypes are classified into class I (HM40), class II (Hβ9), class III (Hβ4), class IVa (H5β), class IVb (Hβ2), class V, and class VI (Hβ1) according to the sequence of the carboxy-terminal domain (Roman numbers denote the protein isotype, and the indication in parentheses shows the human genetic classification.) Class III (Hβ4), i.e. tubulin beta-4B chain (Homo sapiens), is a microtubule-forming protein composed mainly of cytoskeletal proteins and is said to play a role in maintaining cell shape and physiological activities. The tubulin beta-4B chain is a protein consisting of 445 amino acids SEQ ID NO: 1, with a molecular weight of 50255 and a pI of 4.79.

The origin of tubulins contained in the decellularized cellular structure is not limited as long as they are derived from eukaryotes, but mammalian or avian-derived tubulins are preferable. Mammals include cattle, horses, camels, llamas, donkeys, yaks, sheep, pigs, goats, deer, alpacas, dogs, Japanese raccoons, weasels, foxes, cats, rabbits, hamsters, guinea pigs, rats, mice, squirrels, raccoons, or the like. Birds include parakeets, parrots, chickens, ducks, turkeys, geese, guinea fowls, pheasants, ostriches, quails, emus or the like.

Furthermore, the decellularized cellular structure of the present invention can include, without limitation, variants of tubulin, as long as the effects of the present invention is obtained. The tubulin variant include, for example
(1) a polypeptide containing an amino acid sequence in which one or more amino acids (preferably 1 to 10, more preferably 1 to 7, and even more preferably 1 to 5) in total, for example, one to several amino acids in total, are deleted, substituted, inserted, and/or added at one or more locations in the amino acid sequence of tubulin (for example, the amino acid sequence represented by SEQ ID No. 1), and exhibiting tubulin activity, or
(2) a polypeptide having an amino acid sequence having 90% or more homology with the amino acid sequence of tubulin (for example, the amino acid sequence represented by SEQ ID No. 1), and exhibiting tubulin activity.

The activity of tubulin is, for example, to improve the ability to maintain thickness and hardness after decellularization compared to those without the modification, as a decellularized cell structure in this invention

### (Amino acid sequence in which one or more amino acids are deleted, substituted, inserted, and/or added)

The variant may be a polypeptide consisting of an amino acid sequence in which one or more amino acids are deleted, substituted, inserted, and/or added in the amino acid sequence of tubulin (for example SEQ ID No. 1). The polypeptide of variant maintains thickness and hardness after decellularization. In other words, a polypeptide that does not exhibit the ability to maintain thickness and hardness after decellularization is not included in the polypeptide of variant. In this specification, the wording "amino acid sequence in which one or more amino acids are deleted, substituted, inserted, and/or added" means that the amino acid sequence has been modified by amino acid substitutions or the like. The number of amino acid modifications may be 1 to 30, 1 to 20, 1 to 15, 1 to 10, preferably 1 to 8, more preferably 1 to 6, even more preferably 1 to 5, most preferably 1 to 2. Preferably, examples of amino acid sequences of the variants that can be used in the present invention may be amino acid sequences having one or more (preferably, 1, 2, 3 or 4) conservative substitutions of its amino acids.

### (Amino acid sequence having 90% or more identity to amino acid sequence)

The variant may be a polypeptide consisting of an amino acid sequence that has 90% or more identity with the amino acid sequence of tubulin (for example SEQ ID No. 1). The polypeptide of variant maintains thickness and hardness after decellularization. In other words, a polypeptide that does not exhibit the ability to maintain thickness and hardness after decellularization is not included in the polypeptide of variant. The polypeptide comprises an amino acid sequence having the identity is more preferably 95% or more, more preferably 96% or more, more preferably 97% or more, more preferably 98% or more, more preferably 99% or more, and exhibits the ability to maintain thickness and hardness after decellularization.

The "amino acid sequence in which in which one or more amino acids are deleted, substituted, inserted, and/or added" or "amino acid sequence having 90% or more identity to amino acid sequence" in the amino acid sequence of the tubulin (for example, SEQ ID No. 1) is an amino acid sequence of an tubulin (for example, SEQ ID No. 1) is substituted. However,
The substitutions in the amino acid sequence are conservative substitutions that maintain the function of the tubulin used in the present invention. In other words, the "conservative substitution" means a substitution that does not cause loss of the excellent effect of tubulin. That is to say, it is a substitution that maintains thickness and hardness after decellularization even in the case of insertion, substitution, deletion, or addition. Specifically, it means the replacement of an amino acid residue with another chemically similar amino acid residue. For example, as the conservative substitution, there may be mentioned a substitution of a hydrophobic residue for another hydrophobic residue, or a substitution of a polar residue for another polar residue having the same charge. Amino acids which have similar chemical properties and can be conservatively substituted with each other are known to those skilled in the art. More particularly, as nonpolar (hydrophobic) amino acids, there may be mentioned, for example, alanine, valine, isoleucine, leucine, proline, tryptophan, phenylalanine, or methionine. As polar (neutral) amino acids, there may be mentioned, for example, glycine, serine, threonine, tyrosine, glutamine, asparagine, or cysteine. As basic amino acids having a positive charge, there may be mentioned, for example, arginine, histidine, or lysine. As acidic amino acids having a negative charge, there may be mentioned, for example, aspartic acid or glutamic acid.

The above proteins (e.g., tubulin), are microtubule-forming proteins consisting mainly of cytoskeletal proteins, and are assumed to play a role in maintaining the cell shape. In other words, although not limited to this, the function of maintaining cell shape is considered to be related to the maintenance of thickness and hardness after decellularization. Therefore, it is considered that the above proteins (e.g., tubulin) effectively act on the strength of the decellularized cellular structure.

The present disclosure includes the following embodiments:
[1] a decellularized cellular structure, which is a decellularized cultured cellular structure, characterized by having porosity ratio of 30% to 80%,
[2] the decellularized cellular structure of the item [1], wherein a hardness of the decellularized cellular structure is 0.20 to 5.00 (N),
[3] the decellularized cellular structure of the item [1] or [2], wherein a DNA content in dry mass is 0.020 % by weight or less with respect to the decellularized cellular structure,
[4] the decellularized cellular structure of any one of the items [1] to [3], comprising a protein with (a) molecular weight of 45,000 to 53,000 and (b) isoelectric point of pI 4.00 to 4.90,
[5] the decellularized cellular structure of the item [4], wherein the protein is tubulin,
[6] a method for preparing a decellularized cellular structure, comprising: a formation step of cultured cellular structure wherein the cultured cellular structure is formed by culturing cells, and a decellularization step wherein the cultured cellular structure is decellularized to adjust porosity ratio of 30% to 80%,
[7] the method for preparing a decellularized cellular structure of the item [6], wherein the cells are cultured in an any shape before or simultaneously with the formation of the cultured cellular structure in the formation step of cultured cellular structure,
[8] the method for preparing a decellularized cellular structure of the item [7], wherein the culture of cells in the any shape before the formation of the cultured cellular structure in the formation step of cultured cellular structure, is sheet-cell culture or cellular spheroid culture,
[9] the method for preparing a decellularized cellular structure of the item [6], wherein after the formation step of cultured cellular structure, the cells are cultured in an any shape,
[10] the method for preparing a decellularized cellular structure of any one of items [6] to [9], wherein the decellularization is performed by high hydrostatic pressure treatment, and
[11] The method for preparing a decellularized cellular structure of any one of items [6] to [10], wherein a thickness retention ratio before and after decellularization in the decellularization step is 90% or more.

The present invention is not limited to the above embodiments. The above embodiments are examples, and any structures and methods having substantially the same constitutions as the technical concept described in the claims of the present invention and exhibiting the same effect, are included in the technical scope of the present invention.

### EXAMPLES

The present invention will now be further illustrated by, but is by no means limited to, the following Examples.

### «Example 1»

In this example, a decellularized cellular structure was prepared using fibroblasts.

### (Preparation of cultured cellular structure)

NHDF-Neo-dermal fibroblasts (CC-2509, Lonza) were cultured in a T-175 flask (vent cap, 175cm², Violamo). The cells were detached at about 90-100% confluency and cell suspension (10 mL) was prepared (about 2.0×10⁷ cells). A dimple plate was placed in a 10 cm dish, 100% ethanol (10 mL) was added thereto, and ethanol was spread evenly over the entire surface of the dimple plate. After confirming that there were no air bubbles, ethanol was removed. This procedure was repeated twice using phosphate buffer solution (PBS, 0.01M, pH 7.4). To the dimple plate, 10 mL of FKCM medium (serum-free medium for fibroblasts, Fukoku Co., Ltd) was added, and the above cell suspension was added in full and incubated at 37°C in a 5% CO2 incubator (IP400, Yamato Scientific Co., Ltd). After incubation overnight, spheroids (aggregation of cells) in the dimple plates were checked under an inverted microscope (CKX31, Olympus). Spheroids were pipetted to remove them from depressions of the dimple plate and transferred to a 10 cm dish. The 10-cm dish was swirled to collect spheroids in the center of the dish and placed into a net mold (Net Mold Starter Kit V6, TissueByNet). The net mold was transferred to a 60-mL safety container (WATSON) containing FKCM medium (80 mL), and it was placed on a shaker (OS-762, Optima) in a 37°C, 5% CO2 incubator, and then an incubation was started under shaking (45 rpm). The culture was continued by replacing half of the medium once or twice a week. After about 3 weeks of culture, the net mold was removed and a cultured cellular structure was obtained.

The above method for forming the cultured cellular structure shows an example of the method, wherein cells are cultured in an any shape before the formation of the cultured cellular structure in the formation step of cultured cellular structure, and wherein the culture of cells in the any shape in the formation step of cultured cellular structure, is cellular spheroid culture, in the claims. The above method for forming the cultured cellular structure shows an example wherein the cultured cellular structure is formed by maintaining the cellular spheroids in the cavity, and culturing them, and producing extracellular matrix (ECM).

### (Decellularization treatment of cultured cellular structure)

The cultured cell structure removed from the net mold and the FKCM medium were placed in nylon polyethylene vacuum bags (Kyo Bijin, Kurilon Inc.) and it was double-sealed on all four sides with a sealer. The high hydrostatic pressure treatment was carried out using the FKCM medium as a medium, at 600 MPa for 10 minutes using a high-pressure apparatus for research and development (Dr. CHEF, Kobe Steel, Ltd.). The high hydrostatic pressure treatment was carried out at a temperature range of a minimum temperature of 23.3°C and a maximum temperature of 35.9°C. The cultured cellular structure was removed from the nylon polyethylene vacuum bag and transferred into an embedding cassette (TISSUE-TEK), and then immersed in normal saline solution and washed at 4°C for at least 5 minutes with shaking. Subsequently, the cultured cellular structure was immersed in DNase I (e.g. nucleolytic enzyme) solution (800 U/mL) and washed at 4°C for at least 18 hours with shaking. Then, the cultured cellular structure was immersed in normal saline solution and washed three times at 4°C for at least 5 minutes with shaking to obtain decellularized cellular structure.

### «Example 2»

In this example, a decellularized cellular structure was prepared by conducting the high hydrostatic pressure treatment at 200MPa, using fibroblasts.

The procedure described in Example 1 was repeated, except that the high hydrostatic pressure treatment at 200MPa instead of the high hydrostatic pressure treatment at 600MP, to obtain a decellularized cellular structure.

### «Example 3»

In this example, a decellularized cellular structure was prepared by conducting the high hydrostatic pressure treatment at 400MPa, using fibroblasts.

The procedure described in Example 1 was repeated, except that the high hydrostatic pressure treatment at 400MPa instead of the high hydrostatic pressure treatment at 600MP, to obtain a decellularized cellular structure.

### «Example 4»

In this example, a decellularized cellular structure was prepared by freezing and thawing treatment.

The cultured cellular structure removed from the net mold was frozen on dry ice or in a freezer, stored at -80°C for at least 30 minutes, and then thawed in phosphate buffer solution (PBS, 0.01 M, pH 7.4) warmed to 37°C. The cultured cellular structure was then frozen and thawed three times. The cell structures were then immersed in DNase I solution (800 U/mL) of nuclease and washed with shaking for 24 hours at 4°C. The cultured cellular structure was immersed in DNase I (e.g. nucleolytic enzyme) solution (800 U/mL) and washed at 4°C for 24 hours with shaking. Then, the cultured cellular structure was immersed in normal saline solution and washed at 4°C for at least 2 hours with shaking to obtain decellularized cellular structure.

### <<Comparative Example 1>>

In this comparative example, the decellularization treatment was carried out by a surfactant treatment. The cellular structure obtained in Example 1 was decellularized by the following surfactant treatment.

The cultured cellular structure removed from the net mold was washed with injection water at 4°C for 1 hour. Then, the cultured cell structure was treated with 0.25 mass% sodium dodecyl sulfate solution (10 mMTris, pH 8.0) at 4°C for 24 hours. Subsequently, the cultured cell structure was treated with 0.5 mass% Triton-X (polyoxyethylene octylphenyl ether) solution (10 mM Tris, pH 8.0) at 4°C for 24 hours. Then, the cultured cellular structure was immersed in normal saline solution and washed three times at 4°C for at least 5 minutes with shaking to obtain decellularized cellular structures.

### « Calculation of decellularized DNA ratio »

The masses of the decellularized cellular structures of Examples 1 to 4 and Comparative Example 1 were measured, and the samples were immersed in proteolytic enzyme solution to dissolve them. Then, they were treated with phenol/chloroform to remove proteins, and DNAs were recovered. The recovered DNAs were fluorescently stained by Picogreen (Life Technologies) and the DNAs were quantified by measuring the fluorescence intensity. The DNA contents of the samples were calculated from the mass of the samples and the amounts of DNAs. For the quantification of DNA, a calibration curve prepared by using the standard DNA supplied with Picogreen was used. Using samples prepared separately, the dry weight loss ratio was calculated from the mass of the sample (70% moisture content) and the mass of the dried sample (stored in a thermostatic chamber at 60°C for 12 hours). Then, the DNA content per dried mass of the sample was calculated from the DNA content of the sample and the dry weight loss ratio. The results are shown in Table 7. (Decellularized DNA ratio)=(DNA content of dried specimen of decellularized cellular structure)/(mass of dried specimen of decellularized cellular structure)

**Table 1**

| | DNA content(ng) (A) | Mass of specimen(mg) (B) | Decellularized DNA ratio (A/B × 100; mass%) |
|---|---|---|---|
| Example 1 | 87.9 | 6.64 | 0.00132 |
| Example 2 | 178.0 | 9.29 | 0.00192 |
| Example 3 | 137.5 | 8.51 | 0.00162 |
| Example 4 | 166.7 | 9.15 | 0.00182 |
| Comparative Example 1 | 2106.4 | 6.68 | 0.0315 |

### <<Preparation of paraffin-embedded tissue specimens and section staining samples>>

As pretreatment, the decellularized cellular structures obtained in Examples 1 to 4 or Comparative Example 1 were immersed in 4 mass% PFA (4 mass% paraformaldehyde phosphate buffer, for tissue fixation by FUJIFII,M Wako Pure Chemicals Corporation) for at least 48 hours. Then, the decellularized cellular structures were immersed in phosphate buffer solution (PBS, 0.01M, pH 7.4) and washed at 4°C for at least 30 minutes with shaking. Subsequently, the decellularized cellular structures were immersed in 70 mass% ethanol and washed at 4°C for at least 30 minutes with shaking, and they were used as samples for preparation of paraffin-embedded tissue specimens.

Paraffin-embedded tissue specimens were prepared from the above samples according to the following procedure, and section staining samples were prepared for staining (see below). Tissues were rinsed with tap water for 30 to 40 minutes to remove formaldehyde. Samples were dehydrated in an ethanol bath using a paraffin embedding apparatus (CT-Pro20, Genostaff) in the following order:
70 mass% ethanol for 20 minutes (×1)
95 mass% ethanol for 20 minutes (×2)
100 mass% ethanol for 20 minutes (×2)

Further, the samples were washed twice in xylene for 20 minutes each. The samples were incubated twice in a paraffin bath at 65°C for 30 minutes each. A melted paraffin was poured into a mold, the sample was placed in the mold, and allowed to cool for 15 to 20 minutes to prepare a paraffin-embedded tissue specimen. Then, the paraffin-embedded tissue specimens were cut into 5-µm-thick serial sections on a microtome and floated in a 37°C water bath filled with deionized water. The sections were floated one by one in distilled water on a glass slide, warmed on a stretching table (30-40°C), and dried in a constant incubator (37°C) for 1 to 2 nights to make section staining samples.

### (Sirius red staining)

According to the following procedure, the section staining samples were stained by Sirius red staining, to stain the extracellular matrix (ECM).

First, the section staining samples were deparaffinized, hydrated in distilled water, and rinsed with distilled water. Next, the section staining samples were treated with iron hematoxylin (a mixture of equal parts of iron hematoxylin I (1 mass% hematoxylin (SIGMA-ALDRICH)/95 mass% ethanol) and iron hematoxylin II (Mutoh Pure Chemical Co., Ltd)) for 2 minutes and rinsed with distilled water. Then, the samples were treated with 0.5 mass% hydrochloric acid/70 mass% ethanol solution for 3 minutes. Subsequently, the samples were treated with 0.2 mass% phosphorus molybdate solution for 5 minutes. Then, the samples were treated with 0.1 mass% Direct Red80 (Sirius red, SIGMA-ALDRICH)/saturated picric acid solution for 90 minutes.
Subsequently, the samples were dip-treated three times with 0.01N hydrochloric acid. Then, the section staining samples were dehydrated, xylene-dehydrated by permeabilization, and encapsulated with a mounting medium (MountQuick: Daido Sangyo Co., Ltd.).

### «Calculation of porosity ratio»

The obtained Sirius red stained sections were observed with a microscope, and the sections were photographed at 10× magnification, and the porosity ratio of the decellularized cellular structures of Examples 1 to 4 and Comparative example 1 were calculated using image processing software (ImageJ). The images of the section staining samples were captured and the area of the entire decellularized cell structure and the area of the part stained by Sirius red (ECM) were measured, respectively.

The porosity ratio of the decellularized cell structures were calculated according to the following equation. A box area (310µm×310µm) was set as the measurement area, and the average of the porosity ratio of the three areas was calculated. In order to exclude large cavities, a region not containing cavities of 34,000 (about 35%) µm² or more was set. Porosity ratio (%)=(Area of entire decellularized cellular structure (measurement area) - Area stained by Sirius red of Example or Comparative Example) / Area of entire decellularized cellular structure (measurement area) × 100

### « Calculation of thickness retention ratio»

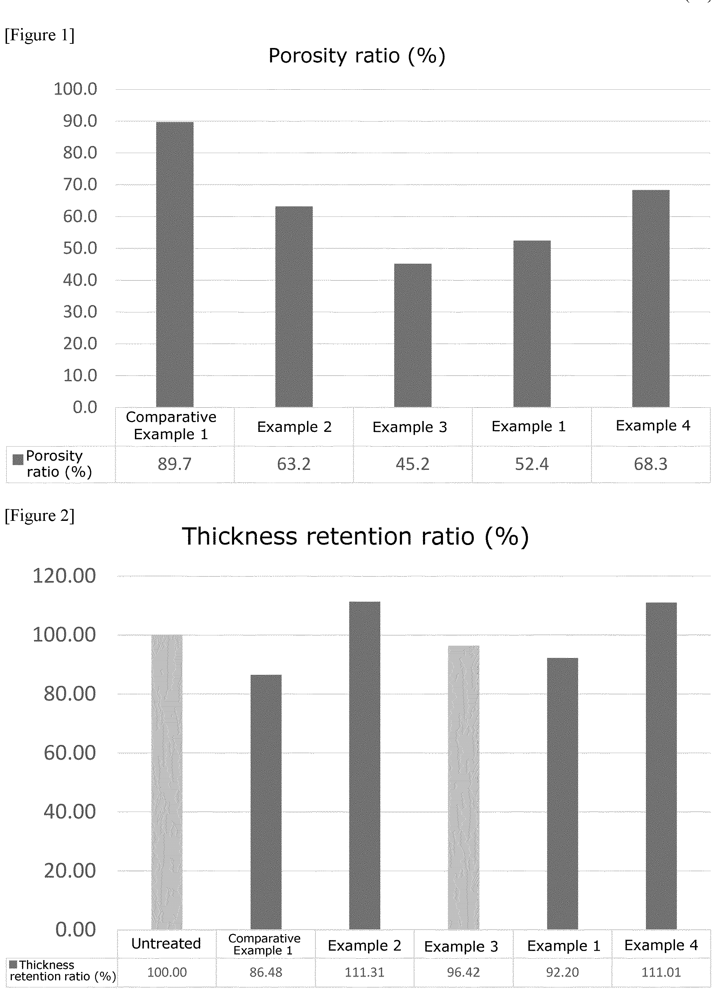

The thickness retention ratios were calculated from the thickness of the cultured cellular structures, and the thicknesses of the decellularized cellular structures of Examples 1 to 4 and Comparative Example 1. The thickness of the cultured cellular structure refers to the "untreated" in Figure 2, which is described later. Thickness retention ratio (%)=(thickness of decellularized cellular structure/thickness of cultured cellular structure) × 100

Figure 1 shows the results of porosity ratios and Figure 2 shows the results of calculation of thickness retention ratios. The porosity ratios of the samples in Examples 1 to 4 ranged from 30 to 80%, while that of the sample in Comparative Example 1 was larger than that of the samples in Examples. As for the thickness retention ratio, it was found that the thickness of the samples after decellularization in Examples 1 to 4 was maintained relative to that before decellularization, and the shapes of the samples before and after decellularization were maintained, compared to that in Comparative Example 1. It was found that, by adjusting the porosity ratio of the decellularized cellular structures to determined values, the shape of the cellular structures before and after decellularization is maintained, and the shape of the decellularized cellular structures can be made into any desired shape.

### <<Hardness test>>

In order to measure the hardness of the decellularized cellular structures obtained in the Examples and Comparative Example, Texture Profile Analysis (TPA) was performed as follows.

TPA was measured at room temperature by a texture analyzer TA.XT plus (EKO INSTRUMENT CO., LTD) equipped with a P/4 cylindrical probe (4 mm in diameter).
The point at which a load of 0.01(N) was applied to the probe was set as the zero point, and the probe was compressed twice on a distance of 0.5mm at a rate of 0.02mm/sec. The maximum force (N) at the first compression cycle indicates the hardness.

The following Manufacturing Examples 1 and 2 were also measured in the same manner.

### (Manufacturing Example 1)

The bovine pericardium was cut open to form a sheet, and the fat was removed entirely (hereinafter, this sheet of bovine pericardium is referred to as "pericardial sheet"). This was used as the pericardial sheet of Manufacturing Example 1.

### (Manufacturing Example 2)

The pericardial sheet of Manufacturing Example 1 was placed in a nylon polyethylene vacuum bag with injection water adding phosphate buffer solution (PBS, 0.01M, pH 7.4) as a medium, and then treated with high hydrostatic pressure at 600 MPa for 10 min using a high-pressure apparatus for research and development (Dr. CHEF, Kobe Steel, Ltd.). The high hydrostatic pressure treated pericardial sheet was treated with nucleolytic enzyme, DNase I (125 U/mL), washed at 4°C for 18 h or more with shaking, then treated in 80% ethanol at 4°C for at least 1 hour, and finally washed with 4 L of injection water at 4°C. After lyophilization, it was irradiated with gamma rays (25 kGy). This was used as the decellularized pericardial sheet (decellularized tissue) of Manufacturing Example 2

The test results are shown in Figure 4, respectively. The hardness of the pericardial sheet of Example 1 was 10.13 (N), and the hardness of the decellularized pericardial sheet (decellularized tissue) of Example 2 was 13.62 (N).

Compared to the Comparative Example, the hardness of the Examples was maintained, indicating that they can be used stably as grafts. In particular, it was found that Example 1 showed good results in terms of stability as a graft. In addition, it was found that Example 1 was softer than the pericardial sheet of Manufacturing Example 1 and the decellularized tissue of Manufacturing Example 2, and has a different hardness from them.

### <<Protein analysis >>

The compositional differences of the decellularized cellular structure of Example 1 and the decellularized cellular structure of Comparative Example 1 were analyzed by two-dimensional electrophoresis.

To the decellularized cell structures of the example and comparative example, 0.3 mL of protein lysis solution (containing urea and surfactant) was added, and the mixture was shaken at 1,400 rpm for 2 hours at room temperature. After homogenization using a Polytron homogenizer, the supernatant was collected by centrifugation at 20,000Xg for 30 minutes at room temperature. The sample solutions were purified using the 2-D Clean-Up Kit (Cytiva, Global Life Science Technologies Japan Inc). Then, protein quantification and electrical conductivity measurements were performed to confirm that the measured values were within the permissible limits for two-dimensional electrophoresis, and the samples were used for two-dimensional electrophoresis.

After adding buffer solution to the samples for two-dimensional electrophoresis, protein lysis solution (containing urea and surfactant) was added to make the total volume of 0.34mL of the sample solutions. The sample solution was placed in a swelling tray, and a precast gel for first dimension electrophoresis (Immobiline DryStrip, Cytiva, Global Life Science Technologies Japan Inc.) was placed on top of the sample solution. A dry strip cover solution (PlusOne DryStrip Cover Fluid, cytiva, Global Life Science Technologies Japan, Inc.) was overlaid and allowed to stand overnight. The swollen precast gels were set on the electrophoresis apparatus (Multiphor II Electrophoresis Unit, cytiva, Global Life Science Technologies Japan, Inc.) and subjected to swimming (500 V for 1 min, 3500 V for 7.5 h, 20°C).

Acrylamide gels with a concentration gradient of 10 to 20% were used. The gels were allowed to stand for 24 hours after preparation to allow the acrylamide to polymerize completely. The equilibrated first dimension electrophoresis precast gel was placed on the acrylamide gel and fixed with 1% agarose solution containing 0.125% bromophenol blue. A molecular weight marker was applied to the left end of the gel to use as a standard for molecular weight. The gel was run at 80 V for 17 hours until the bromophenol blue band was visible at the lower edge of the gel.

### « Protein Staining »

After electrophoresis, gels were stained with fluorescent stain for total protein detection (SYPRO Ruby protein gel stain, S21900, Thermo Fisher Scientific Inc.), and images were saved by a fluorescence scanner. Images were captured by the scanner at 488 nm of excitation wavelength, 640 nm Bandpass of fluorescence filter, 100 micrometer of resolution.

Tiff image files of the obtained fluorescence-stained images were imported into ImageMaster Platinum (Cytiva, Global Life Science Technologies Japan, Inc.) and analyzed numerically. In the numerical analysis, firstly landmarks were manually assigned to the common spots in each gel. After up to 340 spots per gel were landmarked, the gels were matched. By this process, the spots developed on the two gels (gel applied with Example 1 and gel applied with Comparative Example 1) were given common spot IDs. Then, spots of the gels were detected and the spot signal concentrations (%volume) were calculated.

The spot signal concentration was calculated from the ratio of the spot signal with respect to the sum of the signals of all the spots in the gel. The %Volume value is expressed as a percentage, and the minimum value is 0.001%.

The spots that meet the following two conditions were considered as the identified proteins.
- The sum of the spot signal concentrations of the SYPRO Ruby stained images (the sum of two gels when two images are combined) should be 0.1 or more.
- The amount of change of spot signal concentration (the amount of change between gels when two images are combined) should be 0.5 times or less, or 2 times or more.

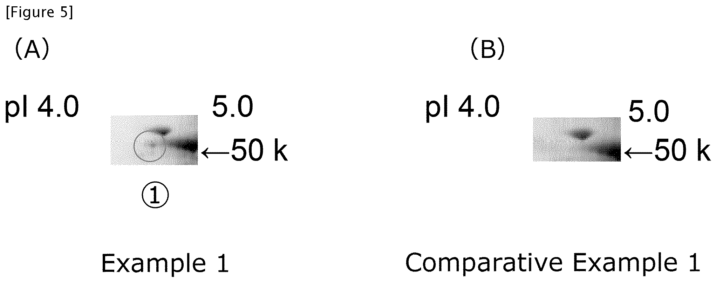

The results of two-dimensional electrophoresis are shown in Figure 5. In Example 1, one spot (1) was detected around the isoelectric point pI4.00 to 4.90 and the molecular weight of 45,000 to 53,000. In contrast, in Comparative example 1, the spot detected in Example 1 were not detected. It was found that Example 1 contains specific protein that is not found in Comparative Example 1.

### « Spot identification »

After the fluorescence-stained images were captured, silver staining was performed using silver nitrate (195-09382, Fujifilm Wako Pure Chemicals Corporation).

The spots visualized by silver staining were cut out, and 100 µL of potassium ferricyanide (15 mM) and 100 µL of sodium thiosulfate (50 mM) were added to a 1 mm square gel piece and shaken for 10 minutes. The solution was discarded, Milli-Q water was added and shaken, and the gel was washed until the color was removed from the gel pieces. Acetonitrile was added to the gel pieces and the gels were dehydrated. The gel pieces were swollen by adding 10 µL of an enzyme solution of 100 mM ammonium bicarbonate and 0.02 µg/µL trypsin, and allowed to stand at 37°C for 16 hours. Peptides were extracted by adding 50 µL of 0.1% TFA and 50% acetonitrile and shaking for 20 minutes. Extraction was performed twice. The peptide extract was concentrated by vacuum centrifugation until the peptide extract was reduced to approximately 10 µL. The samples were adsorbed on ZipTip C18 (millipore, ZTC18S960) and the peptides were eluted with 2.5 µL of 60% acetonitrile and 0.1% TFA solution. One µL of the sample solution was mixed with 1 µL of the CHCA matrix solution, dropped onto the target plate, dried, and then measured by a mass spectrometer (ultrafleXtreme). The obtained mass values were used to identify the proteins in the database (NCBI RefSeq). Based on the obtained mass values, the spots were identified from proteins in the database (NCBI RefSeq).
Analyzer: ultrafleXtreme (Bruker Daltonics)
Target plate: MTP Anchorchip 600/384 (209513, Bruker Daltonics)
Polarity: positive mode
Detection mode: reflector mode (300-6,000 m/z)
CHCA matrix solution: 0.3 g/L CHCA, 33% acetone, 66% ethanol

### MS/MS Ion Search conditions

Identification software: Mascot (Matrix Science)
Database: NCBI RefSeq
Search species: human (Genome assembly GRCh38.p13)
Enzyme: Trypsin
Fixation Modification: Carbamidomethylation

The results of the identification of the spot in Example 1 using the mass spectrometer are shown in Table 2. The spot number in Table 2 below correspond to the three spot numbers in Figure 5. The spot was identified as tubulin.

**[Table 2]**

| Spot number | Protein | molecular weight | Isoelectric point |
|---|---|---|---|
| ① | Tublin beta-4B chain | 50255 | 4.79 |

### INDUSTRIAL APPLICABILITY

The decellularized cellular structures of the present invention can be used as grafts without rejection for the treatment of various diseases.

## Claims

1. A decellularized cellular structure, which is a decellularized cultured cellular structure, **characterized by** having porosity ratio of 30% to 80%.

2. The decellularized cellular structure according to claim 1, wherein a hardness of the decellularized cellular structure is 0.20 to 5.00 (N).

3. The decellularized cellular structure according to claim 1 or 2, wherein a DNA content in dry mass is 0.020 % by weight or less with respect to the decellularized cellular structure.

4. The decellularized cellular structure according to claim 1 or 2, comprising a protein with (a) molecular weight of 45,000 to 53,000 and (b) isoelectric point of pI 4.00 to 4.90.

5. The decellularized cellular structure according to claim 4, wherein the protein is tubulin.

6. A method for preparing a decellularized cellular structure, comprising:
a formation step of cultured cellular structure wherein the cultured cellular structure is formed by culturing cells, and
a decellularization step wherein the cultured cellular structure is decellularized to adjust porosity ratio of 30% to 80%.

7. The method for preparing a decellularized cellular structure according to claim 6, wherein the cells are cultured in an any shape before or simultaneously with the formation of the cultured cellular structure in the formation step of cultured cellular structure.

8. The method for preparing a decellularized cellular structure according to claim 7, wherein the culture of cells in the any shape before the formation of the cultured cellular structure in the formation step of cultured cellular structure, is sheet-cell culture or cellular spheroid culture.

9. The method for preparing a decellularized cellular structure according to claim 6, wherein after the formation step of cultured cellular structure, the cells are cultured in an any shape.

10. The method for preparing a decellularized cellular structure according to any one of claims 6 to 9, wherein the decellularization is performed by high hydrostatic pressure treatment.

11. The method for preparing a decellularized cellular structure according to any one of claims 6 to 9, wherein a thickness retention ratio before and after decellularization in the decellularization step is 90% or more.
